(19) Europäisches Patentamt
European Patent Office
Office européen des brevets

(11) EP 1 382 350 A1

(12) **EUROPEAN PATENT APPLICATION**
published in accordance with Art. 158(3) EPC

(43) Date of publication:
**21.01.2004 Bulletin 2004/04**

(21) Application number: **02722741.2**

(22) Date of filing: **24.04.2002**

(51) Int Cl.[7]: **A61K 45/00**, A61K 38/09, A61K 9/50, A61K 9/52, A61K 47/34, A61P 5/08, A61P 35/00, A61P 43/00

(86) International application number:
**PCT/JP2002/004071**

(87) International publication number:
**WO 2002/087616 (07.11.2002 Gazette 2002/45)**

(84) Designated Contracting States:
**AT BE CH CY DE DK ES FI FR GB GR IE IT LI LU MC NL PT SE TR**
Designated Extension States:
**AL LT LV MK RO SI**

(30) Priority: **25.04.2001 JP 2001128032**

(71) Applicant: **Takeda Chemical Industries, Ltd.**
**Osaka-shi, Osaka 541-0045 (JP)**

(72) Inventors:
- **IGARI, Yasutaka**
  **Kobe-shi, Hyogo 658-0015 (JP)**
- **KUSAKA, Masami**
  **Kobe-shi, Hyogo 651-2102 (JP)**

(74) Representative: **Rickard, Timothy Mark Adrian**
**Takeda Patent Office,**
**11-12 Charles II Street**
**London SW1Y 4QU (GB)**

# (54) PREVENTIVES FOR POSTOPERATIVE RECURRENCE OF PREMENOPAUSAL BREAST CANCER

(57) The agent for preventing the postoperative recurrence of premenopausal breast cancer comprising a GnRH agonist or antagonist of the present invention can prevent the postoperative recurrence of premenopausal breast cancer without severe side effects. In addition, by using the agent of the present invention in the form of a long term sustained-release type microcapsule, the drug action can be maintained for a long time without frequently administering the drug, and thus, prevention of the postoperative recurrence of premenopausal breast cancer having a long recurrence-preventing term and having high convenience becomes possible.

EP 1 382 350 A1

## Description

TECHNICAL FIELD

The present invention relates to (1) an agent for preventing the postoperative recurrence of premenopausal breast cancer comprising a GnRH agonist or antagonist, (2) an agent for preventing the postoperative recurrence of premenopausal breast cancer comprising a sustained-release preparation or an implant preparation containing a GnRH agonist or antagonist, and the like.

BACKGROUND ART

Estrogen, a sex steroid hormone, is considered to be involved in the development or progression of breast cancer responsive to hormone. For treatment of breast cancer, usually, primary lesions are first excised by an operation and then adjuvant therapy is performed for preventing the recurrence. Major adjuvant therapy includes radiation therapy, endocrine therapy and chemical therapy. For endocrine therapy, a drug having a therapeutic effect on the postoperative recurrence or progression of breast cancer has been studied for effectiveness as adjuvant therapy and as a result, tamoxifen (tamoxifen citrate: manufactured by Astrazeneca) which is an anti-estrogen agent has been widely used.

Tamoxifen is an estrogen receptor antagonist and exhibits its efficacy by competing with endogenous estrogen. Therefore, since the endogenous estrogen concentration is higher before menopause where estrogen is synthesized in ovary as compared with after menopause where estrogen is not synthesized in ovary, the efficacy of tamoxifen before menopause is lower than after menopause. In addition, since tamoxifen may act as an estrogen receptor agonist depending on tissues, there is a possibility of promoting the development of endometrial cancer or the like (Journal of National Cancer Institute, Vol. 91, pp 1654-1662).

As described above, although tamoxifen which is an anti-estrogen agent is known as an agent for preventing the postoperative recurrence of premenopausal breast cancer, its effect is decreased before menopause where the endogenous estrogen concentration is higher as compared with after menopause. In addition, a possibility of promoting the development of endometrial cancer or other tumors is pointed out. Then, there is need for development of an agent for preventing postoperative recurrence, which exhibits more potent effect against the postoperative recurrence of premenopausal breast cancer and has no possibility of promoting the development of endometrial cancer or the like.

DISCLOSURE OF THE INVENTION

In view of such situation, we intensively studied in order to develop an agent for preventing the postoperative recurrence of premenopausal breast cancer having no estrogen action and as a result, found out that estrogen can be continuously decreased by administering a GnRH agonist or antagonist (preferably, by administering it as a sustained-release preparation). In addition, we found out that the prolonged interval between administrations of a sustained-release preparation can improve convenience of patients and, based on this, we further studied, which resulted in completion of the present invention.

That is, the present invention provides:

(1) an agent for preventing the postoperative recurrence of premenopausal breast cancer comprising a GnRH agonist or antagonist,
(2) the agent according to the above (1), wherein the GnRH agonist is a peptide represented by the formula:

5-oxo-Pro-His-Trp-Ser-Tyr-Y-Leu-Arg-Pro-Z

wherein Y denotes a residue selected from DLeu, DAla, DTrp, DSer(tBu), D2Nal and DHis(ImBzl), and Z denotes $NH-C_2H_5$ or $Gly-NH_2$, or a salt thereof,
(3) the agent according to the above (1), wherein the GnRH agonist is a peptide represented by the formula:

5-oxo-Pro-His-Trp-Ser-Tyr-DLeu-Leu-Arg-Pro-$NH-C_2H_5$

or a salt thereof,
(4) the agent according to the above (1), wherein the GnRH agonist is acetate of a peptide represented by the formula:

5-oxo-Pro-His-Trp-Ser-Tyr-DLeu-Leu-Arg-Pro-NH-$C_2H_5$,

(5) the agent according to the above (1), which comprises a sustained-release preparation or an implant preparation containing a GnRH agonist or antagonist,
(6) the agent according to the above (5), wherein the sustained-release preparation is a sustained-release type microcapsule,
(7) the agent according to the above (6), wherein the sustained-release type microcapsule is a long term sustained-release type microcapsule which releases a GnRH agonist or antagonist over 2 months or longer,
(8) the agent according to the above (1), which comprises a peptide represented by the formula:

5-oxo-Pro-His-Trp-Ser-Tyr-DLeu-Leu-Arg-Pro-NH-$C_2H_5$

or a salt thereof and a biodegradable polymer,
(9) the agent according to the above (8), wherein the biodegradable polymer is a lactic acid-glycolic acid polymer or polylactic acid,
(10) a method of preventing the postoperative recurrence of premenopausal breast cancer, which comprises administering an effective amount of a GnRH agonist or antagonist to a mammal,
(11) use of a GnRH agonist or antagonist for preparation of an agent for preventing the postoperative recurrence of premenopausal breast cancer,
(12) use of a GnRH agonist or antagonist for preventing the postoperative recurrence of premenopausal breast cancer, and the like.

BEST MODE FOR CARRYING OUT THE INVENTION

Examples of the GnRH agonist or antagonist include GnRH agonists or antagonists effective against hormone dependent diseases, in particular, sex hormone dependent cancers (e.g. prostate cancer, uterus cancer, breast cancer, pituitary tumor etc.), and sex hormone dependent diseases such as prostatic hypertrophy, endometriosis, uterine myoma, precocious puberty, dysmenorrhea, amenorrhea, premenstrual syndrome and polycystic ovary syndrome, and for contraception (or sterility in the case where a rebound effect after stopping administration is utilized). Additional examples of the GnRH agonist or antagonist include GnRH agonists or antagonists effective against benign or malignant tumors or the like which are sex hormone independent, but GnRH sensitive.
Embodiments of the GnRH agonist or antagonist include peptides described in Treatment with GnRH analogs: Controversies and perspectives [published by The Parthenon Publishing Group Ltd. in 1996], JP-A 3-503165, JP-A 3-101695, JP-A 7-97334, JP-A 8-259460, and the like.
As the GnRH antagonist, for example, a peptide represented by the general formula [I]:

X-D2Nal-D4ClPhe-D3Pal-Ser-A-B-Leu-C-Pro-$DAlaNH_2$

wherein X denotes N($4H_2$-furoyl)Gly or NAc, A denotes a residue selected from NMeTyr, Tyr, Aph(Atz) and NMeAph (Atz), B denotes a residue selected from DLys(Nic), DCit, DLys(AzaglyNic), DLys(AzaglyFur), DhArg($Et_2$), DAph(Atz) and DhCi, and C denotes Lys(Nisp), Arg or hArg($Et_2$), or a salt, and Abarelix and the like are used.
In addition, examples of the non-peptide GnRH antagonist include those described in WO 95/28405 (JP-A 8-295693), WO 97/14697 (JP-A 9-169767), WO 97/14682 (JP-A 9-169735), and WO 96/24597 (JP-A 9-169768), thienopyridine compounds, e.g. 3-(N-benzyl-N-methylaminomethyl)4,7-dihydro-5-isobutyryl-7-(2,6-difluorobenzyl)-2-[4-[(1-hydroxycyclopropyl)carbonylamino]phenyl]-4-oxothieno[2,3-b]pyridine (WO 00/00493), thienopyrimidine compounds, e.g. 5-(N-benzyl-N-methylaminomethyl)-1-(2,6-difluorobenzyl)-6-[4-(3-methoxyureido)phenyl]-3-phenylthieno[2,3-d]pyrimidine-2,4(1H, 3H)-dione (WO 00/56739), and 5-[(3,5,5,8,8-pentamethyl-5,6,7,8-tetrahydro-2-naphthalenyl)methyl]-N-(2,4,6-trimethoxyphenyl)-2-furamide (American Association of Cancer Research (AACR), 2002, 4, 6-10).
As the GnRH angonist, for example, a physiologically active peptide represented by the general formula [II]:

5-oxo-Pro-His-Trp-Ser-Tyr-Y-Leu-Arg-Pro-Z

wherein Y denotes a residue selected from DLeu, DAla, DTrp, DSer(tBu), D2Nal and DHis(ImBzl), and Z denotes

$NH-C_2H_5$ or $Gly-NH_2$, or a salt thereof is used. Preferably, the peptide wherein Y is DLeu and Z is $NH-C_2H_5$ or a salt thereof (that is, a peptide represented by 5-oxo-Pro-His-Trp-Ser-Tyr-DLeu-Leu-Arg-Pro-$NH-C_2H_5$ or a salt thereof, in particular, acetate thereof (Leuprorelin acetate: manufactured by Takeda Chemical Industries, Ltd.) is used.

The peptides exemplified as the GnRH agonist or antagonist may be in the form of pharmacologically acceptable salts. Examples of such salts include salts with inorganic acids (e.g. hydrochloric acid, sulfuric acid, nitric acid, boric acid etc.), or organic acids (e.g. carbonic acid, bicarbonic acid, succinic acid, acetic acid, propionic acid, trifluoroacetic acid etc.) when the peptides have a basic group such as an amino group.

When the peptides have an acidic group such as a carboxyl group, examples of such salts include salts with inorganic bases (e.g. alkali metals such as sodium and potassium, alkaline earth metals such as calcium and magnesium, and the like) or organic bases (e.g. organic amines such as triethylamine, basic amino acids such as arginine, and the like). Alternatively, the peptides may form a metal complex compound (e.g. copper complex, zinc complex etc.).

These peptides or salts thereof can be prepared by methods described in the above references or publications or similar methods.

Preferable embodiments of the GnRH agonist in addition to the aforementioned Leuprorelin (Leuprorelin acetate) include:

(1) Buserelin

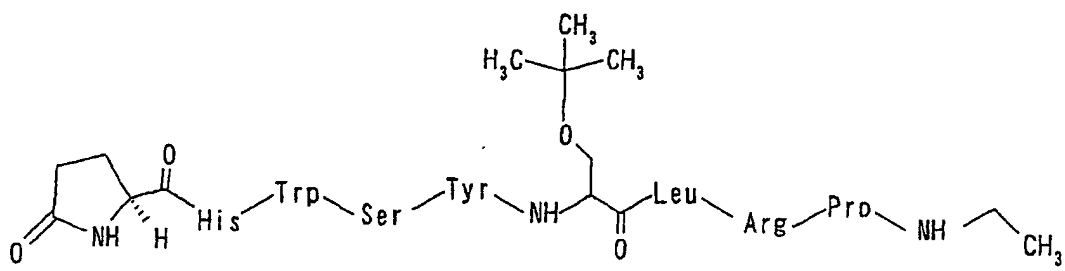

(U.S.P. No. 4,024,248, German Patent No. 2438352, JP-A 51-41359),

(2) Triptorelin

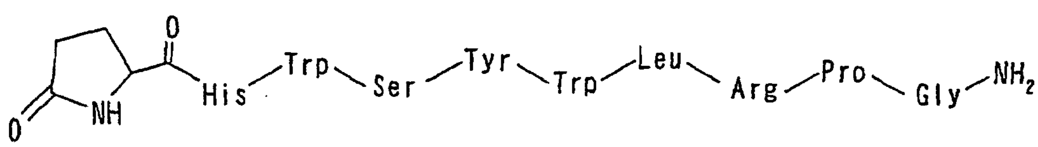

(U.S.P. Publication No. 4010125, JP-A 52-31073),

(3) Nafarelin

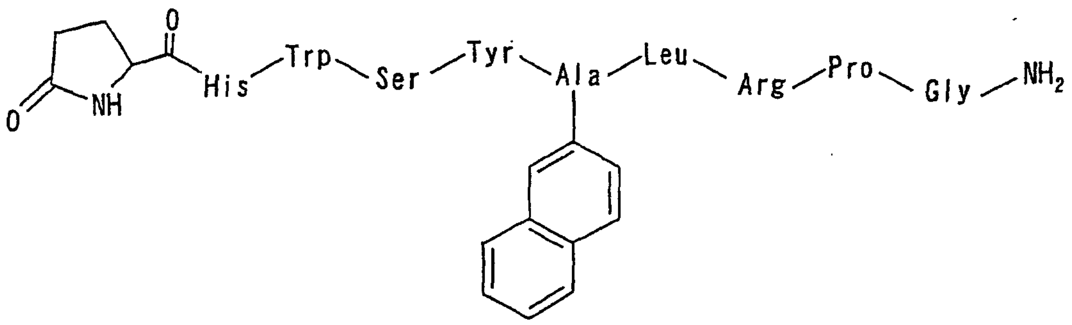

(U.S.P. Publication No. 4234571, JP-A 55-164663, JP-A 63-264498, JP-A 64-25794),

(4) Histrelin

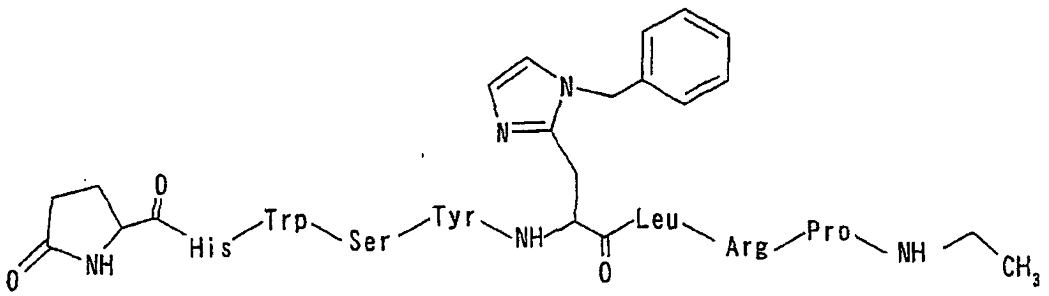

(5) Deslorelin

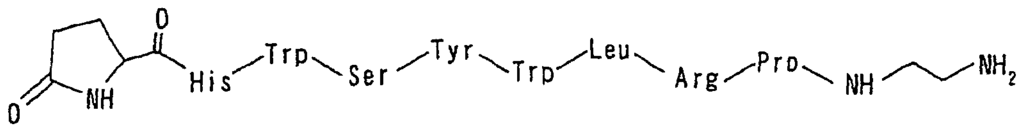

(U.S.P. Publication No. 4569967, U.S.P. Publication No. 4218439),

(6) Meterelin

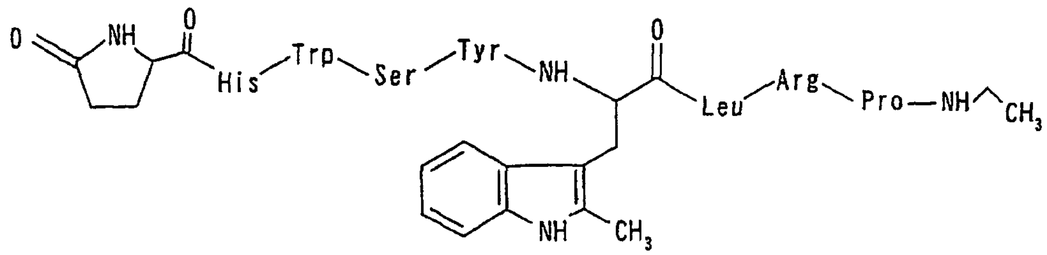

(PCT WO 91/18016),

(7) Gonadrelin

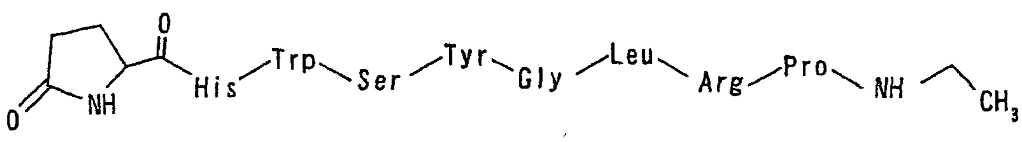

(German Patent No. 2213737) and salts thereof.

In addition, although the following Goserelin is excluded from the GnRH agonists used in the present invention, Goserelin may be used together with the aforementioned GnRH agonists.

(8) Goserelin

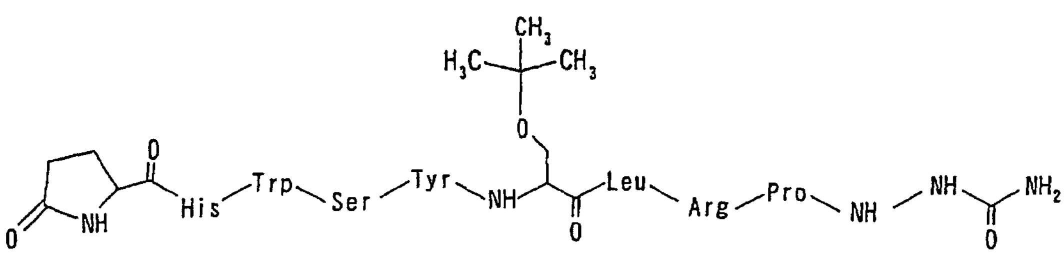

(U.S.P. Publication No. 4100274, JP-A 52-136172).

In addition, the aforementioned GnRH agonists or antagonists (preferably, a peptide represented by the formula: 5-oxo-Pro-His-Trp-Ser-Tyr-DLeu-Leu-Arg-Pro-NH-$C_2H_5$ or a salt thereof (hereinafter, simply referred to as "Leuprorelin or a salt thereof" in some cases)), more preferably Leuprorelin acetate can be administered orally in the dosage form of a tablet optionally covered with sugar, a capsule, an elixir, a sustained-release preparation or the like, or can be administered parenterally in the dosage form of an injection such as a sterile solution or suspension in water or other pharmaceutically acceptable liquid and a sustained-release preparation (in particular, sustained-release microcapsule), an implant preparation (e.g. an implant preparation molded using a biodegradable polymer as a base, an implant preparation obtained by encapsulating an active ingredient into a biocompatible metal tube such as titanium tube so as to release the active ingredient at a constant rate), an injection obtained by dissolving or dispersing a biodegradable polymer and a drug in an organic solvent which can be administered to a living body, or a nasal preparation such as a solution or suspension. The Leuprorelin or a salt thereof is administered preferably as a sustained-release preparation, more preferably as a sustained-release injection. In addition, when the sustained-release preparation is a sustained-release type microcapsule, it is preferably a long term sustained-release type microcapsule which releases a GnRH agonist or antagonist over 2 months or longer.

The aforementioned preparations can be prepared by mixing Leuprorelin or a salt thereof, more preferably Leuprorelin acetate with a known physiologically acceptable carrier, flavor, excipient, vehicle, preservative, stabilizer, binder, etc. in a generally-recognized unit dosage form required for pharmaceutical practice.

The additives for a tablet or a capsule include a binder such as gelatin, corn starch, tragacanth and gum arabic, an excipient such as crystalline cellulose, a swelling agent such as corn starch, gelatin and arginic acid, a lubricant such as magnesium stearate, a sweetener such as sucrose, lactose and saccharin, and a flavor such as peppermint, akamono oil and cherry. When a unit form of the preparation is a capsule, a liquid carrier such as a fat or oil may be added to the aforementioned materials. A sterile composition for injection can be formulated according to a conventional pharmaceutical practice, for example, by dissolving or suspending an active ingredient in a vehicle such as water for injection, naturally occurring vegetable oil such as sesame oil and coconut oil, and the like. As an aqueous liquid for injection, for example, a physiological saline, an isotonic solution containing glucose and other adjuvants (e.g. D-sorbitol, D-mannitol, sodium chloride etc.) or the like is used. Such aqueous liquid for injection may be used together with a suitable solubilizer such as alcohols (e.g. ethanol) and polyalcohols (e.g. propylene glycol, polyethylene glycol), and a nonionic surfactant (e.g. Polysorbate 80 (TM), HCO-50). As an oily liquid for injection, for example, sesame oil, soybean oil or the like is used. Such oily liquid for injection may be used together with a solubilizer such as benzyl benzoate and benzyl alcohol.

In addition, the aforementioned preparations may contain, for example, a buffer (e.g. phosphate buffer, sodium acetate buffer), a soothing agent (e.g. benzalkonium chloride, procaine hydrochloride etc.), a stabilizing agent (e.g. human serum albumin, polyethylene glycol etc.), a preservative (e.g. benzyl alcohol, phenol etc.), an antioxidant and the like. The injection thus prepared is usually filled into a sealed container such as an ample and a vial.

A sustained-release preparation (in particular, sustained-release type microcapsule) containing the aforementioned GnRH agonist or antagonist (preferably, Leuprorelin or a salt thereof, more preferably Leuprorelin acetate) can be prepared by a known method, for example, methods described in JP-A 60-100516, JP-A 62-201816, JP-A 4-321622, JP-A 6-192068, JP-A 9-132524, JP-A 9-221417, JP-A 11-279054 and WO 99/360099.

Among the aforementioned sustained-release preparations, a "long term sustained-release type microcapsule which zero-order releases a physiologically active substance over 2 months or longer" described in JP-A 4-321622 is preferably used.

One example of a process for preparing the aforementioned sustained-release type microcapsule will be described below.

First, the GnRH agonist or antagonist (preferably, Leuprorelin or a salt thereof, more preferably Leuprorelin acetate) is dissolved in water at about 20% to 70% (W/W), preferably 25 to 65 % (W/W), more preferably 35 to 60% (W/W) and, if necessary, a drug-retaining substance such as gelatin and basic amino acid is dissolved or suspended therein to obtain an internal aqueous phase solution.

To the internal aqueous phase solution may be added carbonic acid, acetic acid; oxalic acid, citric acid, phosphoric acid, hydrochloric acid, sodium hydroxide, arginine, lysine or a salt thereof as a pH adjusting agent for retaining the stability and solubility of the GnRH agonist or antagonist (preferably Leuprorelin or a salt thereof, more preferably Leuprorelin acetate). In addition, albumin, gelatin, citric acid, sodium ethylenediaminetetraacetate, dextrin, sodium hydrogen sulfite and a polyol compound such as polyethylene glycol as a stabilizing agent for the GnRH agonist or antagonist (preferably, Leuprorelin or a salt thereof, more preferably Leuprorelin acetate), or parahydoxybenzoic acid esters (methylparaben, propylparaben etc.), benzyl alcohol, chlorobutanol and thimerosal which are generally used as a preservative may be further added.

The internal aqueous phase solution thus obtained is added to a solution (oily phase) containing a polymer, and then emulsified to obtain a W/O type emulsion. The emulsification is performed by a known dispersing methods, for example, an intermittent shaking method, a method using a mixer such as a propeller-type stirrer and a turbine-type stirrer, a colloid mill method, a homogenizer method, and an ultrasound irradiating method.

Then, the W/O type emulsion thus prepared is subjected to a microcapsulation step. As such a step, a dry in water method or a phase separating method may be applied. When a microcapsule is prepared by a dry in water method, the W/O emulsion is further added to an aqueous phase that will form the third phase to form a W/O/W type three phasic emulsion and, thereafter, a solvent in the oily phase is evaporated to prepare a microcapsule.

An emulsifying agent may be added to the aforementioned external phasic aqueous phase. Such an emulsifying agent may be any emulsifying agents which generally form a stable O/W type emulsion, for example, an anionic surfactant (sodium oleate, sodium stearate, sodium laurylsulfate etc.), a nonionic surfactant (polyoxyethylene sorbitan fatty acid ester [Tween 80, Tween 60, Atlas Powder Company], a polyoxyethylene castor oil derivative [HCO-60, HCO-50, Nikko Chemicals] etc.), polyvinylpyrrolidone, polyvinyl alcohol, carboxymethylcellurose, lecithin and gelatin. One

kind of them, or a combination of some of them may be used. The concentration of the emulsifying agent for use can be appropriately selected from the range of about 0.01 % to 20%, more preferably the range of about 0.05 % to 10%.

The evaporation of a solvent from the oily phase is performed by a conventional method. For example, the evaporation is performed by gradually decreasing a pressure with stirring using a propeller-type stirrer or a magnetic stirrer, or by using a rotary evaporator or the like with adjusting a vacuum degree. In such a case, at a point where solidification of a polymer proceeds to an extent, the W/O/W type emulsion is gradually warmed for the purpose of more perfect desorption of a solvent, whereby the required time may be shortened.

The microcapsules thus obtained are collected by centrifugation or filtration. A free GnRH agonist or antagonist (preferably, Leuprorelin or a salt thereof, more preferably Leuprorelin acetate), a drug-retaining substance, an emulsifying agent and the like, which are attached to the surface of the microcapsule, are washed out with distilled water repeatedly a few times. The resulting microcapsules are dispersed again in distilled water, followed by lyophilization. At this time, an aggregation-preventing agent (e.g. mannitol, sorbitol, lactose, glucose etc.) may be added. Removal of moisture and an organic solvent in the microcapsule is performed more perfectly under a reduced pressure, if necessary, with warming.

When a microcapsule is prepared by a phase separating method, a coacervation agent is gradually added to the W/O emulsion under stirring to precipitate and solidify a polymer.

The coacervation agent may be any polymer, mineral oil or vegetable oil compounds which are compatible with a solvent for a polymer and do not dissolve a polymer for encapsulation may be used and, for example, silicone oil, sesame oil, soybean oil, corn oil, cotton seed oil, coconut oil, linseed oil, mineral oil, n-hexane or n-heptane. These coacervation agents may be used in combination of two or more of them.

The microcapsule thus obtained is collected by filtration, and repeatedly washed with heptane or the like to remove a coacervating agent. Further, removal of free drugs and a solvent is performed by the same method as a dry in water method. In order to prevent aggregation of particles during washing, an aggregation-preventing agent may be added.

The microcapsule obtained in the above is sieved after, if necessary, slightly grinding, to remove too large microcapsules. The particle diameter of the microcapsule is in the range of about 0.5 to 1000 μm as an average diameter, more preferably in the range of about 2 to 500 μm. When used as a suspension injection, the particle diameter may be in such the range that the dispersibility and the passage property through a needle are satisfied. For example, the diameter is preferably in the range of about 2 to 100 μm.

As the aforementioned polymer, a biodegradable polymer, for example, a polymer or a copolymer which is synthesized from one or more kinds of α-hydroxycarboxylic acids such as α-hydroxymonocarboxylic acids (e.g. glycolic acid, lactic acid etc.), α-hydroxydicarboxylic acids (e.g. malic acid) and α-hydroxytricarboxylic acids (e.g. citric acid) and has a free carboxyl group, or a mixture thereof; poly(α-cyanoacrylic acid ester); polyamino acid (e.g. poly(γ-benzyl-L-glutamic acid ) etc.); maleic anhydride series copolymer (e.g. styrene-maleic acid copolymer etc.) and the like are used.

A binding form of the monomers may be any of random, block and graft. In addition, when the aforementioned α-hydroxymonocarboxylic acids, α- hydroxydicarboxylic acids and α-hydroxytricarboxylic acids have an optically active center in the molecule, they may be in any form of D-, L- and DL-forms. Among them, lactic acid-glycolic acid polymer [hereinafter, referred to as poly(lactide-co-glycolide), poly(lactic acid-co-glycolic acid) or lactic acid-glycolic acid copolymer and, unless otherwise indicated, collectively referred to a homopolymer (polymer) and a copolymer of lactic acid and glycolic acid, and a lactic acid homopolymer is referred to as lactic acid polymer, polylactic acid or polylactide in some cases, and a glycolic acid homopolymer is also referred to as glycolic acid polymer, polyglycolic acid or polyglycolide], and poly(α- cyanoacrylic acid ester) and the like are preferable. More preferable is a lactic acid-glycolic acid polymer, and further preferable is a lactic acid-glycolic acid polymer having a free carboxyl group at the terminal.

The biodegradable polymer may be a salt. Examples of the salt include salts with inorganic bases (e.g. alkali metals such as sodium and potassium, alkaline earth metals such as calcium and magnesium, etc.) or organic bases (e.g. organic amines such as triethylamine, basic amino acids such as arginine, etc.), and salts and complex salts with transition metals (e.g. zinc, iron, copper etc.).

When a lactic acid-glycolic acid polymer is used as the biodegradable polymer, the composition ratio (mol%) is preferably about 100/0 to about 40/60, more preferably about 100/0 to about 50/50. In addition, in the case of a long term sustained-release type microcapsule which zero-order releases a physiologically active substance over 2 months or longer, a lactic acid homopolymer having the composition ratio of 100/0 is also preferably used.

The optical isomer ratio of lactic acid which is one of minimum repeating units of the "lactic acid-glycolic acid polymer" is preferably in the range of about 75/25 to about 25/75 at the D-form / L-form (mol/mol%) ratio. The lactic acid-glycolic acid polymer wherein the D-form/ L-form (mol/mol%) ratio is in the range of about 60/40 to about 30/70 is particularly generally used.

The weight average molecular weight of the "lactic acid-glycolic acid polymer" is usually about 3,000 to about 100,000, preferably about 3,000 to about 60,000, further preferably about 3,000 to about 50,000.

In addition, the dispersibility (weight average molecular weight/number average molecular weight) is usually preferably about 1.2 to about 4.0, particularly preferably about 1.5 to 3.5.

The amount of a free carboxyl group in the "lactic acid-glycolic acid polymer" is usually preferably about 20 to about 1000 μmol (micromole), particularly preferably about 40 to about 1000 μmol (micromole) per unit mass (gram) of a polymer.

The aforementioned weight average molecular weight, number average molecular weight and dispersibility refer to a molecular weight based on polystyrene which is measured by gel permeation chromatography (GPC) using 15 kinds of monodisperse polystyrenes having a weight average molecular weight of 1,110,000, 707,000, 455,645, 354,000, 189,000, 156,055, 98,900, 66,437, 37,200, 17,100, 9,830, 5,870, 2,500, 1,303 and 504 as a standard substance, and dispersibility calculated therefrom. For the measurement, a high performance GPC apparatus (manufactured by Toso, HLC-8120GPC, the detecting format is by differential refractive index) and GPC column KF804L x 2 (manufactured by Showadenko), and chloroform as a mobile phase are used. The flow rate is 1 ml/min.

The aforementioned amount of a free carboxylic group refers to an amount obtained by a labeling method (hereinafter, referred to as "the amount of carboxyl group by labeling method"). Specifically, the case of polylactic acid will be described. W mg of polylactic acid is dissolved in 2 ml of a mixed solution of 5 N hydrochloric acid/acetonitrile (v/v=4/96). To the solution added are 2 ml of a 0.01 M o-nitrophenylhydrazine hydrochloride (ONPH) solution (5 N hydrochloric acid/acetonitrile/ethanol=1.02/35/15) and 2 ml of a 0.15 M 1-ethyl-3-(3-dimethylaminopropyl)-carbodiimide hydrochloride solution (pyridine/ethanol=4 v/96 v). The mixture react at 40°C for 30 minutes, and then the solvent is distilled off. The residue is washed with water (x4) and then dissolved in 2 ml of acetonitrile, and 1 ml of a 0.5 mol/l ethanolic potassium hydroxide solution is added thereto, followed by reaction at 60°C for 30 minutes. The reaction solution is diluted with a 1.5 N aqueous sodium hydroxide solution to Y ml. The absorbance A (/cm) at 544nm of the dilute solution is measured using a 1.5 N aqueous sodium hydroxide solution as a control. On the other hand, by using an aqueous DL-lactic acid solution as a standard substance, the amount of a free carboxyl group thereof (C mol/l) is determined with alkali titration. In addition, supposing the absorbance at 544 nm of DL-lactic acid hydrazide obtained by the ONPH labeling method is B (/cm), the mole amount of a free carboxyl group per unit mass (gram) of the polymer is determined by the following equation:

$$[COOH]\ (mol/g)=(AYC)/(WB).$$

In addition, the "amount of carboxyl group" may be also obtained by dissolving a biodegradable polymer in a mixed solvent of toluene-acetone-methanol, and titrating a carboxyl group in this solution with an alcoholic potassium hydroxide solution using phenolphthalein as an indicator (hereinafter, a value obtained by such method is referred to as "the amount of a carboxyl group by an alkali titration method"). However, this reaction may compete with a hydrolysis reaction of a polyester main chain during titration and as a result, there is a possibility that a titration endpoint becomes unclear. Therefore, it is desirable to use the aforementioned labeling method for the quantitation.

The "lactic acid-glycolic acid polymer" can be prepared, for example, by catalyst-free dehydration polycondensation from lactic acid and glycolic acid (JP-A 61-28521) or ring-opening polymerization from a cyclic diester compound such as lactide and glycolide using a catalyst (Encyclopedic Handbook of Biomaterials and Bioengineering Part A: Materials, volume 2, Marcel Dekker, Inc. (1995)). A polymer obtained by the aforementioned known ring-opening polymerization method does not necessarily have a free carboxyl group at the terminal. However, the polymer can be converted to a polymer having a carboxyl group to some extent per unit mass, for example, by subjecting to a hydrolysis reaction described in EP-A 0839525, and the polymer thus obtained may be also used.

The aforementioned "lactic acid-glycolic acid polymer having a free carboxyl group at the terminal" can be prepared by the known process (e.g. catalyst-free dehydration polycondensation method, see JP-A 61-28521) or a similar method.

For formulating the microcapsule into an injection, the microcapsule is suspended together with a dispersing agent (e.g. Tween 80, HCO-60, carboxymethylcellulose, sodium alginate etc), a preservative (e.g. methylparaben, propylparaben etc.), an isotonicity agent (e.g. sodium chloride, mannitol, sorbitol, glucose etc.) and the like to obtain an aqueous suspension, or they are dispersed into a vegetable oil such as sesame oil or corn oil to obtain an oily suspension. Thus, an actually usable sustained-release injection is obtained.

Since breast cancer cells are hormone-sensitive and some breast cancer cells grow in response to estrogen, use of a hormone therapeutic drug including Leuprorelin acetate as an agent for preventing the postoperative recurrence of premenopausal breast cancer has been thought to be difficult. However, the agent comprising the GnRH agonist or antagonist (preferably, Leuprorelin or a salt thereof, more preferably Leuprorelin acetate), preferably, the agent comprising the sustained-release type microcapsule containing Leuprorelin or a salt thereof (preferably, Leuprorelin acetate), which is prepared by the aforementioned known method or a similar method, can be unexpectedly used as an agent for preventing or inhibiting the postoperative recurrence of premenopausal breast cancer.

The aforementioned agent for preventing or inhibiting the postoperative recurrence of premenopausal breast cancer comprising the GnRH agonist or antagonist (preferably, Leuprorelin or a salt thereof, more preferably Leuprorelin acetate), preferably, the agent comprising the sustained-release type microcapsule containing Leuprorelin or a salt thereof (preferably, Leuprorelin acetate), can be easily administered subcutaneously, intramuscularly, intravascularly or the like (preferably, subcutaneously) as an injection, implant preparation or the like (preferably injection). Alternatively, the agent of the present invention may be administered in the aforementioned various dosage forms, and may be used as a raw material for manufacture of pharmaceutical preparations with such various dosage forms.

In addition, although the dose of the aforementioned agent for preventing or inhibiting the postoperative recurrence of premenopausal breast cancer varies depending on the content of the GnRH agonist or antagonist (preferably, Leuprorelin or a salt thereof, more preferably Leuprorelin acetate), dosage form, duration time of the GnRH agonist or antagonist (preferably, Leuprorelin or a salt thereof, more preferably Leuprorelin acetate), and subject animals to be administered [e.g. warm-blooded mammals (e.g. human, mouse, rat, rabbit, sheep, pig, bovine, horse)], it may be any effective amount of the GnRH agonist or antagonist (preferably, Leuprorelin or a salt thereof, more preferably Leuprorelin acetate) in preventing or inhibiting the postoperative recurrence of premenopausal breast cancer. For example, the dose per one time for the aforementioned warm-blooded mammal can be appropriately selected from the range of about 0.01 mg to 100 mg/kg weight, preferably about 0.02 mg to 50 mg/kg weight, more preferably 0.05 mg to 20 mg/kg weight.

In addition, when the aforementioned agent for preventing or inhibiting the postoperative recurrence of premenopausal breast cancer is administered as an injection, for an adult patient (body weight 60 kg) after an operation of premenopausal breast cancer, the GnRH agonist or antagonist (preferably, Leuprorelin or a salt thereof, more preferably Leuprorelin acetate) may be subcutaneously or intramuscularly administered in an amount of usually about 0.01 to 50 mg, preferably about 0.1 to 20 mg, more preferably about 0.1 to 15 mg per time. In addition, when the aforementioned agent for preventing or inhibiting the postoperative recurrence comprising the sustained-release type microcapsule containing the GnRH agonist or antagonist (preferably, Leuprorelin or a salt thereof, more preferably Leuprorelin acetate) is administered as an injection, the dose varies depending on a drug sustained-releasing term of the sustained-release type microcapsule. For example, when administered one time per about one mouth to an adult patient (body weight 60 kg) after an operation of premenopausal breast cancer, usually about 0.01 to 20 mg, preferably about 0.1 to 10 mg, more preferably about 0.1 to 5 mg of the GnRH agonist or antagonist (preferably, Leuprorelin or a salt thereof, more preferably Leuprorelin acetate) may be subcutaneously or intramuscularly administered per one time. For example, when administered one time every about three months to an adult patient (body weight 60 kg) after an operation of premenopausal breast cancer, usually about 0.1 to 30 mg, preferably about 0.1 to 20 mg, more preferably about 1 to 15 mg of the GnRH agonist or antagonist (preferably, Leuprorelin or a salt thereof, more preferably Leuprorelin acetate) may be subcutaneously or intramuscularly administered per one time.

The administration term of the agent comprising the GnRH agonist or antagonist (preferably, Leuprorelin or a salt thereof, more preferably Leuprorelin acetate), preferably, the agent comprising the sustained-release type microcapsule containing Leuprorelin or a salt thereof (preferably, Leuprorelin acetate), is not particularly limited, but is usually about for 1 to 5 years, preferably about for 2 years.

In the case of other animals, an amount calculated per weight 60 kg can be administered.

In addition, the GnRH agonist or antagonist (preferably, Leuprorelin or a salt thereof, more preferably Leuprorelin acetate) may be administered as an agent for preventing the postoperative recurrence of premenopausal breast cancer in combination with the following drugs (concomitant drugs).

(1) (Steroid or non-steroid) anti-androgen agents flutamide, casodex, niltamide etc.

(2) (Steroid or non-steroid) anti-estrogen agents

tamoxifen, SERM agent (e.g. raloxifene, arzoxifene, lasofoxifene, TSE-424, SERM-339, SPC-8490), ER down regulator etc.

(3) Chemical therapeutics

ifosfamide, UTF, adriamycin, peplomycin, cisplatin, cyclophosphamide, 5-FU, UFT, methotrexate, mitomycin C, mitoxantrone etc.

(4) 5α-reductase inhibitors

(i) 5α-reductase 2 inhibitors

finasteride, dutasterid, izonsteride, epristeride etc.

(ii) 5α-reductase 1 inhibitors

compounds described in WO93/23420, compounds described in WO95/11254, 4,7β-dimethyl-4-aza-5α-cholestane-3-one, 3-oxo-4-aza-4,7β-dimethyl-16β-(4-chlorophenoxy)-5α-androstane, 3-oxo-4-aza-4,7β-dimethyl-16β-(phenoxy)-5α-androstane etc.

(iii) 5α-reductase 1 and 5α-reducatase 2 double inhibitors

compounds described in WO95/07927, 3-oxo-4-aza-17β-(2,5-trifluoromethylphenyl-carbamoyl)-5α-androstane etc.

(5) α-receptor inhibitors
tamuslosin, prazosin, terazosin, doxazosin, selodosin, alfuzosin etc.
(6) aromatase inhibitors
anastrozole, letrozole, finrozole etc.
(7) 17β-hydroxysteroid dehydrogenase inhibitors
(8) Adrenal androgen production inhibitors
(9) Sex hormone synthesis inhibitors
lyase ($C_{17,20}$-lyase) inhibitors such as abiraterone
(10) Phosphoenzyme inhibitors
(11) Tyrosine phosphoenzyme inhibitors
(12) Hormone therapeutics
corpus luteum hormone agent (e.g. MPA etc.), androgen agent, estrogen agent, growth hormone and derivative thereof, growth hormone secretion promoter etc.
(13) Receptor type tyrosinekinase inhibitors
iressa, imatinib, ODI-774, semaxanib, SU-6668, SU-101, GW-2016, CI-1033 etc.
(14) Side effect alleviators and bone metabolism regulators
bisphosphonate (bisphosphonic acid), bisphosphonate series compounds (e.g. alendronic aicd, etidronic acid, ibandronic acid, incadronic acid etc.), growth hormone secretion promoter (MK-0677), ipriflavone, osteoprotegerin etc.
(15) Immune therapeutics

(15-1) Antibodies, vaccines and immune activators for use in immune therapy specific to prostate (cancer)
PSMA (Prostate-Specific Membrane Antigen) vaccine (Northwest Bio), Dendritic cell therapy (Dendreon), MDX-220 (Medarex), MAB-PSMA (Biovation), Anti-PSM vaccine (M & E Biothech), Prostate cancer vaccine (Corxia), MAb-PSA (AltaRex), MAb-PSMA (Northwestbiotherapeutics), MAb-PSCA (UroGenesys) etc.
(15-2) Other antibodies
131I-chTNT-1/B (Peregrine Pharm), MAB-bispecific-HER2 (Medarex), Cetuximab,
Bevacizumaba (Genentech), SK-1 MAb (Hygeia), PE-40-MAb (BR96, BMS), J-591, Anti-EGFR MAb etc.
(15-3) Other vaccines or immune activators
MAK cells + bispecific antibody (IDM SA), IL-2+cytokines (CEL-SCI), Cancer vaccine (Onyvax), Heat-killed M.vaccae (SR Pharma), GBC-590 (SafeScience), Cancer vaccine (ImmunoTherapy), ADJUVAX-100-A (Jenner), IPS-21 (Biostar), Mycobacterium Cell wall Complex, GPI-0100 (Galenica Pharm), Globo-H-KLM vaccine, BPH therapy (Zonagen), Anti-PSM vaccine (M&E Biothech), Prostate cancer vaccine (Corxia) etc.

(16) EGFR or EGFR antibody and vaccine
cetuximab (IMC C225, ImClone Systems'), EGFR vaccine etc.
(17) T cell differentiation regulators
6,7-dimethoxy-4-(3,4-dimethoxyphenyl)-2-(1,2,4-triazol-1-ylmethyl)quinoline-3-carboxylic acid ethyl ester (JP-A 7-118266) etc.
(18) HER2 antibodies
(19) Cytokine/chemokine inhibitors

(19-1) Protein preparations

(i) TNF inhibitors
etanercept, infliximab, D2E7, CDP-571, PASSTNF-α, soluble TNF receptor, TNF-α binding protein, anti-TNF-α antibody etc.
(ii) Interleukin-1 inhibitors
anakinra (interleukin-1 receptor antagonist), soluble interleukin-1 receptor etc.
(iii) Interleukin-6 inhibitors
MRA (anti-interleukin-6 receptor antibody), anti-interleukin-6 antibody, Sant-7 (interleukin-6 receptor antagonist) etc.
(iv) Interleukin-10 drugs interleukin-10 etc.
(v) Interleukin-12 inhibitors anti-interleukin-12 antibody etc.
(v) Drugs having a combination of interferon-α and -γ inhibition and TNF-α inhibition

AGT-1

(19-2) None-protein preparations

(i) CXCR4 antagonists
(ii) CCR7 antagonists
(iii) MAP kinase inhibitors
PD-98059 etc.
(iv) Gene regulators
inhibitors of molecules involved in signal transmission such as SP-100030, NF-κ, NF-κB, IKK-1, IKK-2 and AP-1, etc.
(v) Cytokine production inhibitors
T-614, SR-31747, sonatimod etc.
(vi) TNF-α converting enzyme inhibitors
(vii) Interleukin-1 β converting enzyme inhibitors
HMR3480/VX-740 etc.
(viiii) Interleukin-6 antagonists
SANT-7 etc.
(ix) Interleukin-8 inhibitors
IL-8 antagonist, CXCR1 and CXCR2 antagonist etc.
(x) Chemokine antagonists
MCP-1 antagonist etc.
(xi) Interleukin-2 receptor antagonists
Denileukin diftitox etc.
(xii) Therapeutic vaccines
TNF-α vaccine etc.
(xiii) Gene therapeutics
Gene therapeutics for the purpose of enhancing the expression of a gene having the anti-inflammatory activity such as interleukin-4, interleukin-10, soluble interleukin-1 receptor, soluble TNF-α receptor and HSV-tk
(xiv) Antisense compounds

ISIS-104838 etc.

(20) Endoserine receptor antagonists atrasentan, YM-598, TA-0201 etc.
(21) Angiotensin II receptor antagonists
(22) Cyclooxygenase inhibitors (COX-I selective inhibitors, COX-II selective inhibitors etc.)
Salicylic acid derivative (e.g. celecoxib, rofecoxib, aspirin), MK-663, valdecoxib, SC-57666, tiracoxib, S-2474, diclofenac, indomethacin, loxoprofen etc.
(23) Bombesin receptor antagonists, anti-bombesin receptor antibodies, ligand-toxin complexes, etc.
(24) Calcitonin receptor antagonista, anti-calcitonin receptor antibodies, ligand-toxin complexes, etc.
(25) Somatostatin receptor antagonists, anti-somatostatin receptor antibodies, ligand-toxin complexes, etc.
(26) Serotonin receptor antagonists, anti-serotonin receptor antibodies, ligand toxin complexes, etc.
(27) GHRH receptor antagonists, anti-GHRH receptor antibodies, ligand-toxin complexes, etc.
(28) Androgen receptor expression enhancers
IL-6 etc.
(29) Androgen receptor expression lowering drugs
(30) Cytokine activity regulators
(31) Cell growth factor activity inhibitors
(32) Others
vascularization inhibitor, CNS drug [e.g. anti-anxiety drug, sleep-introducing agent, schizophrenia therapeutic agent, Perkinson's disease therapeutic agent, anti-dementia drug (e.g. brain circulation improving agent, brain metabolism activator etc.)], depressor, diabetes therapeutic agent, anti-hyperlipemia agent, nutrient (e.g. vitamin supplement etc.), digestion absorption promoter, gastrointestinal drug, etc.

When the GnRH agonist or the GnRH antagonist (hereinafter, GnRH agonist/GnRH antagonist) is used in combination with a concomitant drug, the administration order of the GnRH agonist/GnRH antagonist and the concomitant drug are not limited. The GnRH agonist/GnRH antagonist or a pharmaceutical preparation thereof and a concomitant drug or a pharmaceutical preparation thereof may be simultaneously administered to a subject, or may be individually administered at an interval. The dose of a concomitant drug may be according to the dose which is clinically used, and may be appropriately selected depending on an administration subject, an administration route, a disease, a combination and the like.

The administration form of the GnRH agonist/GnRH antagonist and a concomitant drug is not particularly limited, and it may be any form wherein the GnRH agonist and a concomitant drug are combined when they are administered. Examples of such administration form include (1) administration of a single pharmaceutical preparation obtained by formulating the GnRH agonist/GnRH antagonist and a concomitant drug at the same time, (2) simultaneous administration via the same administration route of two kinds of pharmaceutical preparations obtained by formulating the GnRH agonist/GnRH antagonist and a concomitant drug separately, (3) administration at an interval via the same administration route of two kinds of pharmaceutical preparations obtained by formulating the GnRH agonist and a concomitant drug separately, (4) simultaneous administration via different administration routes of two kinds of pharmaceutical preparations obtained by formulating the GnRH agonist/GnRH antagonist and a concomitant drug separately, and (5) administration at an interval via different administration routes of two kinds of pharmaceutical preparations obtained by formulating the GnRH agonist/GnRH antagonist and a concomitant drug separately (e.g. administration of GnRH agonist/GnRH antagonist and then a concomitant drug, or administration in the reverse order).

As a concomitant drug, an anti-estrogen agent such as tamoxifen is preferable. For example, it is preferable to use the GnRH agonist for two years and tamoxifen for 5 years concomitantly.

By combining the GnRH agonist or the GnRH antagonist with various concomitant drugs, the following excellent effects can be obtained:

(1) The doses of the GnRH agonist/GnRH antagonist and a concomitant drug can be decreased and thereby the side effects can be alleviated, as compared with the case where they are administered alone,
(2) the drug to be used in combination with the GnRH agonist/GnRH antagonist can be selected depending on the condition of a patient (slight/serious),
(3) selection of a concomitant drug having action mechanism different from that of the GnRH agonist/GnRH antagonist makes possible a longer treatment term,
(4) selection of a concomitant drug having action mechanism different from that of the GnRH agonist/GnRH antagonist makes possible a longer duration of the therapeutic effect,
(5) combination use of the GnRH agonist/GnRH antagonist and a concomitant drug results in the synergistic effect,
(6) Add-Back therapy can be applied by using the GnRH agonist in combination with a concomitant drug,
(7) MAB (Maximum androgen blockade) therapy or Maximum estrogen blockade therapy which inhibits all estrogen actions can be applied by using the GnRH agonist combination with a concomitant drug.

The Add-Back therapy refers to a therapeutic method in which, when the level of sex hormone (testosterone, estrogen, estradiol, etc.) in blood is reduced by administration of a GnRH agonist to prevent or treat a disease which is exacerbated depending on the hormone, the hormone or a drug which is regarded as equivalent to the hormone (hereinafter, abbreviated as Add-Back agent in some cases) is administered as an adjuvant in order to alleviate side effect (e.g. reduction in the amount of a bone salt) resulting from the reduced hormone level, that is, the efficacy of the GnRH agonist. It is preferable to administer the Add-Back agent mainly by oral route.

The MAB therapy is a method by which all androgen actions are blocked, and is used as a method for treating prostate cancer. That is, the MAB therapy refers to a combination of surgical orchiectomy for blocking the action of androgen derived from a testis or use of a GnRH agonist and use of antiandrogen for blocking the action of androgen derived from an adrenal gland. Since some breast cancer cells grow in response to androgen, inhibition of the androgen action or synthesis can be therapy.

The maximum estrogen blockade therapy is a method by which all estrogen actions are blocked, under the same idea as that of the MAB therapy. That is, the maximum estrogen blockade therapy refers to a combination of surgical ovariectomy for blocking the action of estrogen derived from an ovary or use of a GnRH agonist and use of an estrogen receptor antagonist for blocking the action of estrogen biosynthesized from adrenal gland-derived androgen.

Further, intermittent therapy using a GnRH agonist and a GnRH antagonist is also possible. For example, after a GnRH agonist or a pharmaceutical preparation (preferably, sustained-release preparation) containing the agonist is administered, a GnRH antagonist or a pharmaceutical preparation (preferably, sustained-release preparation) containing the antagonist is administered within a duration time of the GnRH agonist. An interval for administering a GnRH agonist and a GnRH antagonist or a pharmaceutical preparation containing each of them can be appropriately selected from a duration time of the GnRH agonist, and preferably, it is one day or longer. When a sustained-release preparation containing a GnRH agonist is used, an interval for administering the two drugs can be appropriately selected from a duration time of the GnRH agonist, that is, 7 days or longer and 12 months or shorter, preferably 10 days or

longer and 6 months or shorter, more preferably 14 days or longer and 4 months or shorter.

During necessary therapy term, the two drugs may be repeatedly administered at an administration interval defined by a duration time of a GnRH agonist or a pharmaceutical preparation (preferably, sustained-release preparation) containing it which is administered in advance. A GnRH antagonist or a pharmaceutical preparation (preferably, sustained-release preparation) containing it is used as a drug to be administered finally in a therapy term, but may be successively administered in order to ensure definition of an administration stoppage term.

In the sustained-release preparation, a duration time of its drug efficacy can be measured by a known method such as EIA and RIA or a similar method.

Since administration of a GnRH antagonist or a pharmaceutical preparation (preferably, sustained-release preparation) containing it after administration of a GnRH agonist or a pharmaceutical preparation (preferably, sustained-release preparation) containing it allows the concentration of a sex hormone (e.g. testosterone, LH, FSH, estrogen etc.) to be recovered rapidly after a duration time of the GnRH antagonist, it is possible to define a clear administration stoppage term in the intermittent therapy.

In addition, since use of a GnRH antagonist or a pharmaceutical preparation (preferably, sustained-release preparation) containing it allows the concentration of a sex hormone (e.g. testosterone, LH, FSH, estrogen etc.) in a living body to be recovered rapidly, it is possible to keep sex hormone dependent disease hormone dependent.

After the sex hormone concentration is recovered, a GnRH agonist or a pharmaceutical preparation containing it and/or a GnRH antagonist or a pharmaceutical preparation containing it can be administered.

For administration of the aforementioned agent for preventing the postoperative recurrence of premenopausal breast cancer, the following requirements should be complied: a primary lesion is proved histopathologically to be breast cancer; there is histopathological or clinical objective evidence that a metastasis lesion or recurrence lesion is breast cancer; a metastasis lesion or recurrence lesion can be measured or assessed, and the measurement or assessment is performed within 4 weeks before initiation of administration of the aforementioned preventing agent; in the case of a soft part tissue such as skin, subcutaneous part or lymph node, there is a finding by a histopathological or cytological diagnosis; in the case of a lesion such as bone or lung, there is an objective finding by various imaging diagnoses (simple X-ray, CT, MRI, ultrasound etc.); and Estrogen Receptor (ER) or Progesterone Receptor (PgR) of a primary lesion or a metastasis lesion or a recurrence lesion is positive, and the like. However, a finding of ER or PgR is not an essential requirement.

In addition, it is also required that a patient has a menses within one year before initiation of the administration, but in the case of a hysterectomized patient, it is required that retention of the ovary function (specifically, both of E2 $\geq$ 30 pg/ml, FSH $\leq$ 50 pg/mL or 20 mU/mL should be satisfied) is only confirmed within one year before initiation of the administration. These requirements are principles, and may be changed with academic progression. Timing of initiating administration of the agent for preventing the postoperative recurrence of premenopausal breast cancer is not particularly limited so long as the aforementioned requirements are satisfied, and it may be any time after an operation such as removal of a breast cancer lesion site of a patient with premenopausal breast cancer. In addition, the administration may be initiated before an operation and continued till after the operation.

Regarding an amino acid, a peptide, a protecting group and the like in polypeptides described herein, when they are expressed by an abbreviation, the expressions are based on abbreviations according to IUPAC-IUB Commission on Biochemical Nomenclature or conventional abbreviations in the art. In addition, when there may be an optical isomer regarding amino acids, L-form is shown unless otherwise indicated.

Examples of abbreviations are shown below:

| | |
|---|---|
| Abu | :Aaminobutyric acid |
| Aibu | :2-Aminobutyric acid |
| Ala | :Alanine |
| Arg | :Arginine |
| Gly | :Glycine |
| His | :Histidine |
| Ile | :Isoleucine |
| Leu | :Leucine |
| Met | :Methionine |
| Nle | :Norleucine |
| Nval | :Norvaline |
| Phe | :Phenylalanine |
| Phg | :Phenylglycine |
| Pro | :Proline |
| (Pyr)Glu | :Pyroglutamic acid |
| Ser | :Serine |

| | |
|---|---|
| Thr | :Threonine |
| Trp | :Tryptophan |
| Tyr | :Tyrosine |
| Val | :Valine |
| D2Nal | :D-3-(2-naphtyl)alanine residue |
| DSer(tBu) | :O-tert-butyl-D-serine |
| DHis(ImBzl) | : $N^{im}$-benzyl-D-histidine |
| PAM | :Phenylacetamidomethyl |
| Boc | :t-Butyloxycarbonyl |
| Fmoc | :9-fluorenylmethyloxycarbonyl |
| Cl-Z | :2-Chloro-benzyloxycarbonyl |
| Br-Z | :2-Bromo-benzyloxycarbonyl |
| Bzl | :Benzyl |
| $Cl_2$-Bzl | :2,6-Dichlorobenzyl |
| Tos | :p-Toluenesulfonyl |
| HONb | :N-hydroxy-5-norbornene-2,3-dicarboxyimide |
| HOBt | :1-Hydroxybenzotriazole |
| HOOBt | :3-Hydroxy-3,4-dihydro-4-oxo-1,2,3-benzotriazine |
| MeBzl | :4-Methylbenzyl |
| Bom | :Benzyloxymethyl |
| Bum | :t-Butoxymethyl |
| Trt | :Trityl |
| DNP | :Dinitrophenyl |
| DCC | :N,N'-dicyclohexylcarbodiimide |

Examples

The following Examples illustrate the present invention in detail.

Example 1

To a postoperative patient with premenopausal breast cancer, Lupron Depot (registered trademark) 11.25 mg (manufactured by TAP Pharmaceutical Products Inc.) is subcutaneously administered at an interval of 3 months 8 times. After 24 months of the initial administration, the recurrence of a lesion location, and the metastasis and recurrence at the contralateral breast in the postoperative patient with premenopausal breast cancer are confirmed by histological (cytological) finding or X-ray photographs. In addition, distant metastasis is confirmed by a conventional method such as an imaging test method and histological or cytological finding.

The breast cancer recurrence or metastasis was hardly found in the postoperative patient with premenopausal breast cancer after 24 months of the initial administration of Lupron Depot (registered trademark) 11.25mg (manufactured by TAP Pharmaceutical Products Inc.). Therefore, Lupron Depot (registered trademark) 11.25mg (manufactured by TAP Pharmaceutical Products Inc.) has preventive effect on the postoperative recurrence of premenopausal breast cancer.

Example 2

To a postoperative patient with premeopausal breast cancer, Leuplin (registered trademark) for injection 3.75 (manufactured by Takeda Chemical Industries, Ltd.) is subcutaneously administered at an interval of 4 weeks 6 times. The estradiol concentration in blood is measured for 24 weeks from initiation of the administration. On the other hand, Lupron Depot (registered trademark) 11.25 mg (manufactured by TAP Pharmaceutical products Inc.) as described in Example 1 is subcutaneously administered to a postoperative patient with premenopausal breast cancer at an interval of 12 weeks 2 times. The estradiol concentration in blood is measured for 24 weeks from initiation of the administration. When changes in the blood estradiol concentrations of the patient administered Leuplin (registered trademark) for injection 3.75 (manufactured by Takeda Chemical Industries, Ltd.) and the patient administered Lupron Depot (registered trademark) 11.25 mg (manufactured by TAP Pharmaceutical Products Inc.) were compared, the both inhibited the blood estradiol concentration to the menopause level similarly. Therefore, Leuplin (registered trademark) for injection 3.75 (manufactured by Takeda Chemical Industries, Ltd.) has preventive effect on the postoperative recurrence of premenopausal breast cancer, like Lupron Depot (registered trademark) 11.25 mg (manufactured by TAP pharmaceutical products Inc.).

Example 3

[Subject patient]

Age: 49 years

Symptom: The patient was diagnosed to be with breast cancer (tumor diameter 1.6 cm) around June, 2001, and was subjected to breast-preserving treatment on July 4 in the same year. As a result of tests during and after the operation, a pathological diagnosed name was mucinous carcinoma, the metastasis to a lymph node was not found and, regarding a hormone receptor, it was confirmed that an estrogen receptor was positive.

The present experiment was explained to the patient on July 18 in the same year. The consent was obtained on August 10 in the same year and administration of Leuplin (registered trademark) for injection 3.75 (manufactured by Takeda Chemical Industries, Ltd.) as described in Example 2 was initiated from the same date. Thereafter, the drug was administered every 4 weeks. It was confirmed that the recurrence of breast cancer was not found from an observation and various imaging diagnoses such as lung X-ray, liver CT and bone scintigram at 24 weeks (January 25, 2002) after the initiation of administration. At present, the administration is being continued, and the administration for 2 years is scheduled.

Example 4

[Subject patient]

Age: 42 years

Symptom: The patient was diagnosed to be with breast cancer (tumor diameter 2.5 cm) around July, 2001, and was subjected to pectoralis-preserving mammectomy (Auchincloss method) on August 23 in the same year. As a result of tests during and after the operation, a pathological diagnosed name was solid duct carcinoma, and the metastasis to a lymph node was not found, and regarding a hormone receptor, it was confirmed that both an estrogen receptor and a progesterone receptor were positive.

The present experiment was explained to the patient on September 13 in same year. The consent was obtained on September 14 in the same year, and administration of Lupron Depot (registered trademark) 11.25 mg (manufactured by TAP Pharmaceutical Products Inc.) as described in Example 1 was initiated from October 3 in the same year. Thereafter, the drug was administered every 12 weeks. It was confirmed that the recurrence of breast cancer was not found from an observation and various imaging diagnoses such as lung X-ray and liver echogram at 24 weeks (March 20, 2002) after the initiation of administration. At present, the administration is being continued, and the administration for 2 years is scheduled.

INDUSTRIAL APPLICABILITY

The agent for preventing the postoperative recurrence of premenopausal breast cancer comprising a GnRH agonist or antagonist of the present invention can prevent the postoperative recurrence of premenopausal breast cancer without severe side effects. In addition, by using the agent of the present invention in the form of a long term sustained-release type microcapsule, the drug action can be maintained for a long time without frequently administering the drug, and thus, prevention of the postoperative recurrence of premenopausal breast cancer having a long recurrence-preventing term and having high convenience becomes possible.

## Claims

1. An agent for preventing the postoperative recurrence of premenopausal breast cancer comprising a GnRH agonist or antagonist.

2. The agent according to claim 1, wherein the GnRH agonist is a peptide represented by the formula:

5-oxo-Pro-His-Trp-Ser-Tyr-Y-Leu-Arg-Pro-Z

wherein Y denotes a residue selected from DLeu, DAla, DTrp, DSer(tBu), D2Nal and DHis(ImBzl), and Z denotes $NH-C_2H_5$ or $Gly-NH_2$, or a salt thereof.

**3.** The agent according to claim 1, wherein the GnRH agonist is a peptide represented by the formula:

5-oxo-Pro-His-Trp-Ser-Tyr-DLeu-Leu-Arg-Pro-NH-$C_2H_5$

or a salt thereof.

**4.** The agent according to claim 1, wherein the GnRH agonist is acetate of a peptide represented by the formula:

5-oxo-Pro-His-Trp-Ser-Tyr-DLeu-Leu-Arg-Pro-NH-$C_2H_5$.

**5.** The agent according to claim 1, which comprises a sustained-release preparation or an implant preparation containing a GnRH agonist or antagonist.

**6.** The agent according to claim 5, wherein the sustained-release preparation is a sustained-release type microcapsule.

**7.** The agent according to claim 6, wherein the sustained-release type microcapsule is a long term sustained-release type microcapsule which releases a GnRH agonist or antagonist over 2 months or longer.

**8.** The agent according to claim 1, which comprises a peptide represented by the formula:

5-oxo-Pro-His-Trp-Ser-Tyr-DLeu-Leu-Arg-Pro-NH-$C_2H_5$

or a salt thereof and a biodegradable polymer.

**9.** The agent according to claim 8, wherein the biodegradable polymer is a lactic acid-glycolic acid polymer or polylactic acid polymer.

**10.** A method of preventing the postoperative recurrence of premenopausal breast cancer, which comprises administering an effective amount of a GnRH agonist or antagonist to a mammal.

**11.** Use of a GnRH agonist or antagonist for preparation of an agent for preventing the postoperative recurrence of premenopausal breast cancer.

**12.** Use of a GnRH agonist or antagonist for preventing the postoperative recurrence of premenopausal breast cancer.

## INTERNATIONAL SEARCH REPORT

International application No.
PCT/JP02/04071

**A. CLASSIFICATION OF SUBJECT MATTER**

Int.Cl[7] A61K45/00, 38/09, 9/50, 9/52, 47/34, A61P5/08, 35/00, 43/00

According to International Patent Classification (IPC) or to both national classification and IPC

**B. FIELDS SEARCHED**

Minimum documentation searched (classification system followed by classification symbols)

Int.Cl[7] A61K45/00, 38/09, 9/50, 9/52, 47/34, A61P5/08, 35/00, 43/00

Documentation searched other than minimum documentation to the extent that such documents are included in the fields searched

| | | | |
|---|---|---|---|
| Jitsuyo Shinan Koho | 1940-1992 | Toroku Jitsuyo Shinan Koho | 1994-1996 |
| Kokai Jitsuyo Shinan Koho | 1971-1992 | Jitsuyo Shinan Toroku Koho | 1996-2002 |

Electronic data base consulted during the international search (name of data base and, where practicable, search terms used)

**C. DOCUMENTS CONSIDERED TO BE RELEVANT**

| Category* | Citation of document, with indication, where appropriate, of the relevant passages | Relevant to claim No. |
|---|---|---|
| X | WO 00/020034 A1 (Takeda Chemical Industries, Ltd.), 13 April, 2000 (13.04.00), Whole document & JP 2000-178202 A & EP 1136079 A1 | 1-9,11 |
| X | WO 98/32423 A1 (Takeda Chemical Industries, Ltd.), 30 July, 1998 (30.07.98), Whole document & JP 10-273447 A | 1-9,11 |
| X | WO 99/36099 A1 (Takeda Chemical Industries, Ltd.), 22 July, 1999 (22.07.99), Whole document & EP 1048301 A1 & JP 11-269094 A | 1-9,11 |

[X] Further documents are listed in the continuation of Box C.　　[ ] See patent family annex.

* Special categories of cited documents:
"A" document defining the general state of the art which is not considered to be of particular relevance
"E" earlier document but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| 15 July, 2002 (15.07.02) | 30 July, 2002 (30.07.02) |
| Name and mailing address of the ISA/ Japanese Patent Office | Authorized officer |
| Facsimile No. | Telephone No. |

Form PCT/ISA/210 (second sheet) (July 1998)

**INTERNATIONAL SEARCH REPORT**

International application No.
PCT/JP02/04071

C (Continuation). DOCUMENTS CONSIDERED TO BE RELEVANT

| Category* | Citation of document, with indication, where appropriate, of the relevant passages | Relevant to claim No. |
|---|---|---|
| X | WO 01/005380 A1 (Takeda Chemical Industries, Ltd.), 25 January, 2001 (25.01.01), Whole document & JP 2001-081043 A & EP 1197208 A1 | 1-9,11 |
| X | EP 839525 A1 (Takeda Chemical Industries, Ltd.), 06 May, 1998 (06.05.98), Whole document & JP 10-182496 A & US 6113943 A | 1-9,11 |

Form PCT/ISA/210 (continuation of second sheet) (July 1998)

## INTERNATIONAL SEARCH REPORT

International application No.
PCT/JP02/04071

**Box I Observations where certain claims were found unsearchable (Continuation of item 1 of first sheet)**

This international search report has not been established in respect of certain claims under Article 17(2)(a) for the following reasons:

1. [X] Claims Nos.: 10, 12
because they relate to subject matter not required to be searched by this Authority, namely:
These claims pertain to methods for treatment of the human body by therapy and thus relate to a subject matter which this International Searching Authority is not required, under the provisions of Article 17(2)(a)(i) of the PCT and Rule 39.1(iV) of the Regulations under the PCT, to search.

2. [ ] Claims Nos.:
because they relate to parts of the international application that do not comply with the prescribed requirements to such an extent that no meaningful international search can be carried out, specifically:

3. [ ] Claims Nos.:
because they are dependent claims and are not drafted in accordance with the second and third sentences of Rule 6.4(a).

***Box II Observations where unity of invention is lacking (Continuation of item 2 of first sheet)***

This International Searching Authority found multiple inventions in this international application, as follows:

1. [ ] As all required additional search fees were timely paid by the applicant, this international search report covers all searchable claims.

2. [ ] As all searchable claims could be searched without effort justifying an additional fee, this Authority did not invite payment of any additional fee.

3. [ ] As only some of the required additional search fees were timely paid by the applicant, this international search report covers only those claims for which fees were paid, specifically claims Nos.:

4. [ ] No required additional search fees were timely paid by the applicant. Consequently, this international search report is restricted to the invention first mentioned in the claims; it is covered by claims Nos.:

**Remark on Protest** [ ] The additional search fees were accompanied by the applicant's protest.
[ ] No protest accompanied the payment of additional search fees.

Form PCT/ISA/210 (continuation of first sheet (1)) (July 1998)